# EUROPÄISCHE PATENTSCHRIFT

(11) **EP 1 401 455 B1**
(45) Veröffentlichungstag und Bekanntmachung des Hinweises auf die Patenterteilung: **23.02.2005**
(21) Anmeldenummer: 02747384.2
(22) Anmeldetag: 12.06.2002
(51) Int. Cl.: A61K 33/26, A61P 19/00, A61P 9/10

(54) **VERWENDUNG EINES PHOSPHATADSORBERS GEGEN GEFÄSSERKRANKUNGEN**
USE OF A PHOSPHATE ADSORBENT TO TREAT VASCULAR DISEASES
UTILISATION D'UN ADSORBANT DE PHOSPHATE POUR TRAITER LES MALADIES VASCULAIRES

(30) Priorität: 13.06.2001 DE 10128511
(43) Veröffentlichungstag der Anmeldung: 31.03.2004
(73) Patentinhaber: SeBo GmbH, 64711 Erbach (DE)
(72) Erfinder: BOOS, Karl-Siegfried, 82131 Gauting (DE); SEIDEL, Dietrich, 82340 Feldafing (DE)
(74) Vertreter: Weiss, Wolfgang, Dipl.-Chem. Dr.
(86) Internationale Anmeldenummer: PCT/EP2002/006465
(87) Internationale Veröffentlichungsnummer: WO 2002/100419

(56) Entgegenhaltungen:
- WO-A-01/15720
- DE-A- 4 239 442
- BLOCK G A ET AL: "Association of serum phosphorus and calcium x phosphate product with mortality risk in chronic hemodialysis patients: a national study." AMERICAN JOURNAL OF KIDNEY DISEASES: THE OFFICIAL JOURNAL OF THE NATIONAL KIDNEY FOUNDATION. UNITED STATES APR 1998, Bd. 31, Nr. 4, April 1998 (1998-04), Seiten 607-617, XP001105475 ISSN: 1523-6838
- BLOCK G A: "Control of serum phosphorus: implications for coronary artery calcification and calcific uremic arteriolopathy (calciphylaxis)." CURRENT OPINION IN NEPHROLOGY AND HYPERTENSION. ENGLAND NOV 2001, Bd. 10, Nr. 6, November 2001 (2001-11), Seiten 741-747, XP001105476 ISSN: 1062-4821

## Beschreibung

Die Erfindung betrifft die Verwendung eines mit polynuklearen Metalloxidhydroxiden modifizierten Adsorptionsmaterial zur Beeinflussung des Calciumspiegels und insbesondere zur Herstellung eines Medikaments zur Behandlung oder/und Vorbeugung von atherosklerotischen Gefäßerkrankungen oder/und Störungen des Knochenstoffwechsels.

Klinische Studien (z.B. G.A. Block et al., Am. J. Kidney Dis. 31 (1998), 607-617) an Dialysepatienten zeigen, dass mit steigendem Calcium-Phosphat-Produkt im Serum dieser Patienten das relative Risiko zu sterben zunimmt, da erhöhte Werte des Calcium-Phosphat-Produkts die Calcifizierung der Blutgefäße, insbesondere der Herzkranzgefäße, vorantreiben.

Eine Erniedrigung des Calcium-Phosphat-Produktes könnte daher insbesondere bei Dialysepatienten die Überlebensrate erhöhen.

Als Therapeutika zur Verringerung der Phosphatkonzentration werden bisher bevorzugt peroral verabreichbare Phosphatbinder eingesetzt, die die Resorption der Nahrungsphosphate im Gastrointestinaltrakt verhindern sollen. Bekannte Substanzen mit phosphatbindenden Eigenschaften sind Calciumsalze (z.B: Calciumacetat, Calciumcarbonat, Calciumcitrat, Calciumalginat, Calciumgluconat, Calciumlactat und Calciumsulfat), Magnesiumcarbonat und Magnesiumhydroxid sowie Aluminiumhydroxid und Aluminiumcarbonat.

Außer diesen salzartigen Phosphatbindern ist aus DE 28 15 811 C2 (1978) ein makroporöses Sorptionsmittel bekannt, das dadurch gekennzeichnet ist, dass es ein organischer Kationenaustauscher ist, der mit lonen mindestens eines Metalls beladen ist, dessen Phosphat nur schwer löslich ist.

Weiterhin beschreibt Burt, H.M. et al. (J. Pharm. Sci. 75 (1978), 379-983) Anionenaustauscher auf DOWEX®-Basis, die als funktionelle Gruppe tertiäre oder quartäre Amine tragen und anorganisches Phosphat im Intestinaltrakt adsorbieren.

Diese Mittel zeigen jedoch erhebliche Nebenwirkungen dahingehend, dass sich der Calciumgehalt im Serum von Patienten erhöht, so dass trotz einer gewissen Verringerung der Phosphatkonzentration nur eine relativ bescheidene Reduzierung des Calcium-Phosphat-Produkts erreicht wird.

Die deutsche Patentanmeldung 42 39 442.2 beschreibt die Verwendung eines mit polynuklearen Metalloxidhydroxiden modifizierten Adsorptionsmaterials zur selektiven Elimination von anorganischem Phosphat aus proteinhaltigen Flüssigkeiten, insbesondere Körperflüssigkeiten. Das Mittel eignet sich daher hervorragend zur Behandlung der Hyperphosphatämie. Ein Einfluss auf die Calciumkonzentration wird jedoch nicht berichtet.

DE 42 39 442 A offenbart ein Verfahren zur Elimination von anorganischem Phosphat aus einer biologischen Flüssigkeit wie z.B. Blut durch Verwendung von mit Metalloxidhydroxiden modifizierten Adsorptionsmaterialien. Diese Materialien können sowohl oral als auch extrakorporal eingesetzt werden

BLOCK G A ET AL, AMERICAN JOURNAL OF KIDNEY DISEASES, 31(4), 1998, 607-617, stellt den Zusammenhang zwischen einem hohen Calcium-Phosphat Produkt und Krankheiten wie Osteodystrophie und vaskulärer Verkalkung dar. Es erwähnt auch, welche Nebenwirkungen die herkommlichen Methoden zur Kontrolle des Phosphatspiegels in Dialysepatienten haben können.

WO 01 15720 A stellt den Zusammenhang zwischen einem hohen Phosphatspiegel und Krankheiten wie Osteodystrophie oder vaskulärer Verkalkung und die Bedeutung der Kontrolle des Phosphatinhaltes im Blut von Dialysepatienten dar.

Überraschenderweise wurde gefunden, dass das mit polynuklearen Metalloxidhydroxiden modifizierte Adsorptionsmaterial im Gegensatz zu bislang verwendeten Phosphatbindern das Calcium-Phosphat-Produkt signifikant, z.B. nach 28tägiger Therapiedauer, um nahezu ein Drittel verringert.

Ein Gegenstand der Erfindung ist somit die Verwendung eines mit polynuklearen Metalloxidhydroxiden modifizierten Adsorptionsmaterials zur Herstellung eines Medikaments zur Behandlung oder/und Vorbeugung von atherosklerotischen Gefäßerkrankungen oder/und Störungen des Knochenstoffwechsels.

Ein weiterer Gegenstand der Erfindung ist die Verwendung eines mit polynuklearen Metalloxidhydroxiden modifizierten Adsorptionsmaterials zur Herstellung eines Medikaments zur Verringerung des Calcium-Phosphat-Produkts im Blut bzw. Serum.

In einer bevorzugten Ausführungsform wird das erfindungsmäße Mittel bei Patienten mit eingeschränkter Nierenfunktion, insbesondere Prädialysepatienten, eingesetzt. Durch Verabreichung kann eine zum Teil erhebliche Verlängerung des prädialytischen Stadiums erreicht werden. In einer weiteren bevorzugten Ausführungsform wird das Mittel an Dialysepatienten verabreicht.

Durch die Verabreichung wird eine Verringerung der Phosphatkonzentration unter gleichzeitiger Beibehaltung - oder in vielen Fällen - sogar Verringerung der Calciumkonzentration erreicht. Auf diese Weise gelingt es, eine Calcifizierung der Blutgefäße von Patienten, insbesondere der Herzkranzgefäße, zu verhindern und somit die Überlebensrate zu erhöhen.

Die mit polynuklearen Metalloxidhydroxiden modifizierten Adsorptionsmaterialien werden vorzugsweise in Form von pharmazeutischen Präparaten eingesetzt, die den Wirkstoff in einer im Wesentlichen unter physiologischen Bedingungen nicht resorptionsfähigen Form enthalten. Die Herstellung solcher unlöslicher Adsorptionsmaterialien ist ausführlich in der deutschen Patentanmeldung 42 39 442.2 beschrieben, auf deren Offenbarung hiermit Bezug genommen wird. Besonders bevorzugt erfolgt die Herstellung derart, dass das unmodifizierte Adsorptionsmaterial mit einer Eisensalzlösung, z.B. Eisen-(III)-chlorid, in Kontakt gebracht und anschließend mit Base, z.B. Natronlauge, auf einen pH-Wert von > 10, insbesondere > 12 eingestellt wird. Das resultierende Präzipitat wird anschließend - gegebenenfalls nach einer Alterung - gespült und kann vor dem Einsatz, wenn erforderlich, nochmals sterilisiert werden.

Die Adsorptionsmaterialien werden vorzugsweise aus löslichen oder unlöslichen Materialen mit organischen oder/und anorganischen Hydroxyl-Funktionalitäten ausgewählt. So können beispielsweise organische Träger, wie etwa natürliche, halbsynthetische oder synthetische, lineare oder verzweigtkettige, lösliche oder unlösliche Kohlenhydrate, organische Polymerisate oder Copolymerisate, z.B. Agarose, Dextran, Dextrin, Stärke, Amylose, Amylopektin, Cellulose oder/und Polyvinylalkohol, als Basismaterial verwendet werden. Andererseits kann man auch anorganische Träger, insbesondere solche auf Siliciumdioxid- oder/und Silicatbasis, wie etwa Glyceryl-modfizierte Gläser und Glyceryl-modifizierte Kieselgele einsetzen. Besonders bevorzugte Ausgangsmaterialien sind Kohlenhydrate, wie etwa Dextrane.

Zur Verwendung als polynukleare Metalloxidhydroxide ist eine große Anzahl von Metallen geeignet, beispielsweise alle Übergangsmetalle, wie etwa Zirkonium, aber auch Aluminium. Als besonders bevorzugtes Metall wird jedoch Eisen verwendet, da bei einer möglicherweise geringeren Ablösung des Metalls Eisen dasjenige Metall ist, das für den Körper als am unschädlichsten anzusehen ist. Aus physiologschen Gründen ist daher dreiwertiges Eisen als Metall am meisten bevorzugt, obgleich auch andere Metalle aufgrund ihrer Bindungseigenschaften bezüglich des anorganischen Phosphats brauchbar sind.

Die erfindungsgemäß verwendeten Adsorptionsmaterialien zeichnen sich dadurch aus, dass sie das polynukleare, kovalent bzw. koordinativ an den Träger gebundene Metalloxidhydroxid bzw. Metallion, insbesondere die bevorzugterweise verwendete Eisen(III)verbindung, auch bei Kontakt mit proteinhaltigen Flüssigkeiten, wie beispielsweise Vollblut und/oder Plasma, nicht in nennenswertem Umfang freisetzen und somit bei der erfindungsgemäßen therapeutischen extrakorporalen oder/und peroralen Anwendung keine unerwünschten Nebenwirkungen, wie beispielsweise eine Störung der enteralen Eisenresorption oder des zellulären und insbesondere des erythrozytären Eisenstoffwechsels, hervorrufen.

Die Verabreichung der Präparate kann auf jede geeignete Art und Weise erfolgen. In einer bevorzugten Ausführungsform werden die Präparate oral für enterale Adsorption bzw. Elimination von anorganischem Phosphat oder/und Calcium verabreicht. Die oralen Präparate können in Form von Pulvern, Tabletten, Kapseln etc., gegebenenfalls überzogen mit einer Magensäure-resistenten Schicht, verabreicht werden. Andererseits können die erfindungsgemäßen Materialien auch in einem extrakorporalen Perfusionssystem zur Behandlung von Vollblut, Plasma oder/und Dialysierflüssigkeit eingesetzt werden.

In einer besonders bevorzugten Ausführungsform kann dabei ein bereits mit Phosphat vorbehandeltes Adsorptionsmaterial eingesetzt werden, welches durch Inkontaktbringen des aus der deutschen Patentanmeldung 42 39 442.2 bekannten Mittels mit einer Phosphat enthaltenden Flüssigkeit, insbesondere einer wässrigen Phosphatlösung, hergestellt werden kann. Dieser Phosphat-vorbehandelte Adsorber eignet sich besonders zur selektiven Verringerung des Calciumgehalts und wird daher bevorzugt, gegebenenfalls zusammen mit einem nicht vorbehandelten Phosphatadsorber, für orale Anwendungen eingesetzt.

Da das Mittel physiologisch verträglich ist und weitgehend nicht resorbiert wird, kann die täglich Behandlungsdosis im Wesentlichen beliebig gewählt werden. So können beispielsweise ohne Weiteres 2-20 g oder sogar mehr des Präparats täglich über einen längeren Zeitraum verabreicht werden.

Die Behandlung ist vorzugsweise eine Langzeitbehandlung und wird für einen Zeitraum von mindestens 6 Monaten durchgeführt, um ihre optimale Wirkung zu entfalten.

Weiterhin soll die Erfindung durch das nachfolgende Beispiel erläutert werden. Bei dem in dem in diesem Beispiel verwendeten polynuklearen Eisen-III-oxidhydroxid-Kohlenhydrat-Komplex handelt es sich um den gemäß Beispiel 1 der deutschen Patentanmeldung 42 39 442 hergestellte Präparat.

### Beispiel:

Im Serum von 13 Dialysepatienten, die mit gängigen Phosphatbindern (Calciumcarbonat, Calciumacetat, Phosphonorm, Renagel®) therapiert wurden, wurde zunächst der Gehalt (Mittelwert) an Phosphat (2,1 mmol/l) und Calcium (2,5 mmol/l) bestimmt und daraus das Calcium-Phosphat-Produkt (5,25) berechnet.

Danach wurden die Phosphatbinder für 7 Tage abgesetzt und der Gehalt an Phosphat (2,9 mmol/l) und Calcium (2,2 mmol/l) erneut im Serum bestimmt und daraus das Calcium-Phosphat-Produkt (6,38) errechnet.

Danach erfolgte die Therapie mit dem erfindungsgemäßen Phosphatbinder (polynuklearer Fe(III)oxidhydroxid-Kohlenhydrat-Komplex) bei einer täglichen Dosis von zunächst 4,6 g Trockengewicht über einen Zeitraum von 14 Tagen und sodann bei einer Dosis von 8,6 g für weitere 14 Tage.

Nach 28tägiger Therapiedauer betrug der Gehalt an Phosphat 1,8 mmol/l und an Calcium 2,1 mmol/l. Das Calcium-Phosphat-Produkt wurde mit 3,78 berechnet.

### Ergebnis

Gegenüber den bislang verwendeten Phosphatbindern wurde bei Verwendung des erfindungsgemäßen Phosphatbinders das Calcium-Phosphat-Produkt überraschenderweise um nahezu ein Drittel erniedrigt.

## Patentansprüche

1. Verwendung eines mit polynuklearen Metalloxidhydroxiden modifizierten Adsorptionsmaterials zur Herstellung eines Medikaments zur Behandlung oder/und Vorbeugung von atherosklerotischen Gefäßerkrankungen oder/und Störungen des Knochenstoffwechsels.

2. Verwendung nach Anspruch 1 zur Verringerung des Calciumphosphatprodukts im Blut.

3. Verwendung nach Anspruch 1 oder 2 zur Verringerung der Phosphatkonzentration unter gleichzeitiger Beibehaltung oder Verringerung der Calcium-Konzentration.

4. Verwendung nach einem der Ansprüche 1 bis 3,
**dadurch gekennzeichnet,**
**dass** das Metalloxidhydroxid ein Eisen-(III)-metalloxidhydroxid ist.

5. Verwendung nach einem der Ansprüche 1 bis 4,
**dadurch gekennzeichnet,**
**dass** das Adsorptionsmaterial aus Kohlenhydraten ausgewählt wird.

6. Verwendung nach einem der Ansprüche 1 bis 5,
**dadurch gekennzeichnet,**
**dass** man ein mit Phosphat vorbehandeltes Adsorptionsmaterial einsetzt.

7. Verwendung nach einem der Ansprüche 1 bis 5 zur oralen Verabreichung.

8. Verwendung nach einem der Ansprüche 1 bis 7 zur extrakorporalen Blutbehandlung.

9. Verwendung nach einem der Ansprüche 1 bis 8 an Patienten mit eingeschränkter Nierenfunktion, insbesondere Prädialysepatienten.

10. Verwendung nach Anspruch 9 zur Verlängerung des prädialytischen Stadiums.

11. Verwendung nach einem der Ansprüche 1 bis 8 an Dialysepatienten.

12. Pharmazeutische Zusammensetzung auf Basis eines mit polynuklearen Metalloxidhydroxiden modifizierten Adsorptionsmaterials, welches mit Phosphat vorbehandelt worden ist.

13. Zusammensetzung nach Anspruch 12,
**dadurch gekennzeichnet,**
**dass** sie eine oral verabreichbare Zusammensetzung ist.

## Claims

1. Use of an adsorption material which has been modified with polynuclear metal oxide hydroxides for producing a medicament for treating and/or preventing atherosclerotic vascular diseases and/or disturbances of bone metabolism.

2. Use according to Claim 1 for reducing the calcium phosphate product in the blood.

3. Use according to Claim 1 or 2 for decreasing the concentration of phosphate while at the same time maintaining or reducing the concentration of calcium.

4. Use according to one of Claims 1 to 3,
**characterized**
**in that** the metal oxide hydroxide is an iron (III) metal oxide hydroxide.

5. Use according to one of Claims 1 to 4,
**characterized**
**in that** the adsorption material is selected from carbohydrates.

6. Use according to one of Claims 1 to 5,
**characterized**
**in that** a phosphate-pretreated adsorption material is employed.

7. Use according to one of Claims 1 to 5 for oral administration.

8. Use according to one of Claims 1 to 7 for extracorporeal blood treatment.

9. Use according to one of Claims 1 to 8 on patients suffering from restricted renal function, in particular predialysis patients.

10. Use according to Claim 9 for prolonging the predialytic stage.

11. Use according to one of Claims 1 to 8 on dialysis patients.

12. Pharmaceutical composition which is based on an adsorption material which has been modified with polynuclear metal oxide hydroxides and which has been pretreated with phosphate.

13. Composition according to Claim 12,
**characterized**
**in that** it is a composition which can be administered orally.

## Revendications

1. Utilisation d'un matériau d'adsorption modifié avec des oxydes-hydroxydes métalliques polynucléaires pour la préparation d'un médicament destiné au traitement et/ou à la prévention de maladies vasculaires athérosclérotiques et/ou de troubles du métabolisme osseux.

2. Utilisation selon la revendication 1 pour la réduction du produit phosphate de calcium dans le sang.

3. Utilisation selon la revendication 1 ou 2 pour la réduction de la concentration de phosphate avec, en même temps, le maintien ou la réduction de la concentration de calcium.

4. Utilisation selon l'une des revendications 1 à 3, **caractérisée en ce que** l'oxyde-hydroxyde métallique est un oxyde-hydroxyde métallique de fer(III).

5. Utilisation selon l'une des revendications 1 à 4, **caractérisée en ce que** le matériau d'adsorption est choisi parmi des hydrates de carbone.

6. Utilisation selon l'une des revendications 1 à 5, **caractérisée en ce que** l'on utilise un matériau d'adsorption prétraité avec du phosphate.

7. Utilisation selon l'une des revendications 1 à 5, pour l'administration orale.

8. Utilisation selon l'une des revendications 1 à 7, pour le traitement extracorporel du sang.

9. Utilisation selon l'une des revendications 1 à 8, chez des patients ayant une fonction rénale limitée, en particulier des patients en prédialyse.

10. Utilisation selon la revendication 9, pour prolonger le stade de la prédialyse.

11. Utilisation selon l'une des revendications 1 à 8, chez des patients en dialyse.

12. Composition pharmaceutique à base d'un matériau d'adsorption modifié avec des oxydes-hydroxydes métalliques polynucléaires qui a été prétraité avec du phosphate.

13. Composition selon la revendication 12, **caractérisée en ce qu'**elle est une composition administrable par voie orale.
